# EUROPEAN PATENT APPLICATION

(11) **EP 1 479 295 A1**
(43) Date of publication of application: **24.11.2004**
(21) Application number: 04011659.2
(22) Date of filing: 17.05.2004
(51) Int. Cl.: A01N 35/06, A01N 41/04, A61K 31/122, C09D 5/14

(54) **Use of naphtoquinone compounds for the preparation of biocidal compositions**

(30) Priority: 20.05.2003 IT mi20031011; 22.12.2003 US 740396; 05.04.2004 IT mi20040678
(71) Applicant: Vanetta S.P.A., 20123 Milano (IT)
(72) Inventor: Manzotti, Paolo, 20052 Monza (Prov. of Milano) (IT); Monteleone, Francesco, 20060 S. Agata di Cassina de'Pecchi (IT)
(74) Representative: Modiano, Guido, Dr.-Ing.

(57) **Abstract**

The biocidal use of quinone compounds similar to menadione in the prevention of development or eradication of the presence of autotrophic or heterotrophic microorganisms in masses of water, preferably ballast water in marine practice, or in animals. The use in water provides for a simple mixing of a series of compounds into the mass of liquid to be treated, whereas if one intends to use the biocidal properties of the described compounds in the zootechnical field, the compounds are incorporated in medicinal compositions, which are then administered to the animals to be treated.

## Description

The present invention relates to the use of one or more quinone compounds to prepare biocidal compositions meant for administration to animals or for the treatment of masses of water to prevent the development, or eradicate the presence, of unwanted autotrophic or heterotrophic microorganisms.

Currently, there are apparently highly dissimilar sectors, such as the maritime sector and the zootechnical sector, which however are deeply united by the need to combat the development of unwanted microorganisms by resorting to biocidal compositions and substances that perform their activity safely and effectively against a broad spectrum of autotrophic and heterotrophic organisms.

For example, in the merchant marine, fresh or salt water is usually taken on board as ballast and is pumped into the bilge of the ship to control its maneuverability, stability and waterline; this water can be taken on board or discharged in various locations along the route of the ship as well as, of course, at the departure and destination ports. It is estimated that the amount of ballast water transferred each year is approximately 10-15000 million tons, and any adult or larva of plankton that is in the vicinity of the drawing point may be picked up and discharged in the subsequent destination ports. Accordingly, over a period of a few years hundreds of autotrophic or heterotrophic organisms can be transferred from one region of the Earth to another, from one continent to another, producing a swift biological invasion, with negative consequences on the host ecosystem.

One of the most vivid examples of this invasion is the settling and abnormal growth, in coastal areas of the United States, of a non-indigenous bivalve, the zebra mussel (hereafter *Dreissena Polimorfa),* which originated in the Asian continent and reached the Americas almost certainly by means of the ballast water of freight ships. Colonies of this bivalve can clog the cooling water pipes of electric power stations or of other industrial facilities and drinking water intake pipes. Moreover, these colonies can interfere with the distribution of phytoplankton and promote the growth of certain blue-green algae (such as for example Microcystis), which are toxic for fish and human beings (as reported in detail in NOAA 1996).

As regards the pharmaceutical sector, particularly applied to the zootechnical field, it is now well-known that human health is currently seriously threatened by the indiscriminate use of conventional antibiotics to raise heavier (fatter) animals, ensuring the economic and production performance of the farm. The indiscriminate use of antibiotics in fact stimulates the emergence of resistant bacterial strains, and this phenomenon can be further amplified by the well-known mechanism of cross-resistance. Moreover, since the microbial ecosystems that are present in animals and human beings are interdependent, resistance to an antibiotic can cross the species barrier and transfer rapidly from animals to humans. Should this resistance transfer to highly dangerous organisms, such as *Staphylococcus aureus,* a bacterium that is already resistant to many antibiotics at present and for which glycopeptides are the last resource, the results could be disastrous.

From the above description it is therefore clear that biocidal substances are widely used in marine practice and in zootechny, since both are based on the need to combat the presence and development of microorganisms that are essentially identical or in any case similar while avoiding risks to man and to the environment and to useful species. This is confirmed by the fact that patent literature describes the use of organic molecules with a biocidal function in both of these sectors.

For example, US 6,164,244 teaches the use of a natural aromatic substance that is normally present in low concentration in the shell of walnuts *(Juglans Regia)* and is commonly known as juglone (5-hydroxy-1,4-naphthalenedione) for the specific treatment of ballast water. According to this patent, juglone has an effective biocidal action for controlling many organisms that can be present in ballast water. However, the use of juglone remains constrained by the need to work around the problem of its extremely low solubility in water. Moreover, it is known (from US 3,602,194) that juglone can be used as a piscicide to eliminate unwanted fish populations in inland waters, but this is a disadvantage when one has to unload into the sea ballast water treated with this compound.

In US 6,340,468, the properties of juglone have been extended to other compounds such as benzoquinones, naphthoquinones and anthraquinones and their derivatives. This document reveals a close link between activity and the presence of at least one hydroxyl group as well as an evident but unexplainable variability in effectiveness on the most feared organisms. Moreover, the problem of limited water solubility of the active ingredient remains, and attempts have been made to remedy this problem by using organic solvents, which however are often toxic. Moreover, the degradability of juglone into harmless by-products is closely dependent on its photoexposure and does not always occur rapidly enough.

In the veterinary field, the biocidal molecules that have been most favoured by farmers are Monensin sodium and Carbadox. However, the former has been found to be toxic for some important farming species, while the latter has been withdrawn from the market because some of its metabolites had a significant carcinogenic activity. The same fate befell other drugs having a coccidiostatic action, when the European Community banned them from the zootechnical field by 2005 (EC Regulation 1831/2003).

Even more recent solutions, such as the massive use of acidifying feed additives described for example in WO96/35337, at present do not seem to be a solution and to be capable of ensuring an adequate protection of animals in field conditions. Furthermore, this last solution has proved itself absolutely inactive against non-bacterial microorganisms, such as protozoa or helminths, which however cause widespread veterinary diseases.

Accordingly, there is a strong need to find substances that have a strong biocidal activity against autotrophic and heterotrophic microorganisms, so that they can be used in the most disparate fields and can perform this task effectively, safely and selectively.

Therefore, the aim of the present invention is to provide a compound whose use as a biocide prevents the growth, or eradicates the presence, of microorganisms, preferably but not exclusively in the zootechnical and marine field, without having the drawbacks of currently known solutions.

Within the scope of this aim, an object of the invention is to provide the biocidal use of a compound that is applied in particular to the zootechnical field to protect economic and production performance in the farming of economically important animal species without thereby entailing risks for human health.

Another object is to provide a composition and the use thereof comprising said compound, to be used preferably but not exclusively as an antimicrobial means in zootechny.

Another object is to provide the biocidal use of a compound that is preferably applied to fresh or salt water in reservoirs of any size, water of water pipes for civil and industrial use, ballast water and aquariums of any size, to control and inhibit the growth, and eliminate the presence, of unwanted autotrophic and heterotrophic species, and wherein the compound is active against a broad spectrum of said autotrophic and heterotrophic species.

Another object is to provide the biocidal use as described above of a compound that is highly water-soluble, easily biodegradable and not harmful for the environment into which it is released at the end of its active cycle.

This aim and these and other objects are achieved by the use of at least one quinone compound selected from the group that consists of menadione, derivatives and mixtures thereof for the production of a biocidal composition for preventing the growth, or eradicating the presence, of at least one microorganism from a substrate selected between a mass of water and an animal,
provided that when the use occurs in a mass of water, the at least one quinone compound is not menadione, menadione sodium bisulfite (MSB), menadione piperazine bisulfite, menadione styramine bisulfite, menadione epoxide, menadione triamino triazine bisulfite, and mixtures thereof.

In a first embodiment, the present invention describes the use of at least one quinone compound other than menadione, menadione sodium bisulfite (MSB), menadione piperazine bisulfite, menadione styramine bisulfite, menadione epoxide, menadione triamino triazine bisulfite, and mixtures thereof to produce a biocidal composition for preventing the growth, or eliminating the presence, of at least one infesting aquatic organism from a mass of water at risk of contamination or already contaminated by said aquatic organism, and wherein said aquatic organism is preferably selected from the group that consists of phytoplankton organisms, dinoflagellates, diatoms, zooplankton organisms and bacteria.

The biocidal composition to be added to the mass of water comprises preferably at least one quinone compound selected from the group that consists of menadione nicotinamide bisulfite (MNB), menadione dimethylpirymidinol bisulfite (MPB), menadione nicotinic acid bisulfite, menadione adenine bisulfite, menadione tryptophan bisulfite, menadione histidine bisulfite, menadione para aminobenzoic acid bisulfite and mixtures thereof, advantageously menadione nicotinamide bisulfite (MNB), even more preferably the composition consists of only one or more of the active molecules cited above.

Preferably, the treated phytoplankton organisms are algae and the zooplankton organisms are selected from the group that consists of copepods, crustaceans, polychaetes, mollusks and their larvae.

The treated bacterial aquatic organisms are both Gram-positive and Gram-negative bacteria. Preferably, they are bacteria that belong to the *Enterobacteriaceae, Pseudomonaceae, Alteromonadaceae* and *Bacillaceae* families. More preferably, the treated bacteria belong to the *Escherichia, Salmonella, Pseudomonas, Alteromonas* and *Clostridium* genera. Preferably treated species are *Escherichia, Salmonella, Pseudomonas, Alteromonas, Clostridium, Shigella* and *Proteus.*

Preferably, the infesting aquatic organisms are selected from the group comprising *Dreissena Polimorfa, Isochris Galbana, Eurytemora Affinis, Tigriopus Fulvus, Artemia salina.*

In a second embodiment, the present invention describes the use of a compound selected from the group that consists of menadione and derivatives and mixtures thereof for the preparation of a medicament for the prevention and treatment, in an animal, of morbid conditions associated with an abnormal proliferation of pathogenic microorganisms.

In a third embodiment, the present invention describes a composition having antimicrobial activity particularly for veterinary use, comprising a compound selected from the group that consists of menadione and derivatives and mixtures thereof, said compound being present in a total quantity comprised between 10 ppm and 250000 ppm by weight on the total weight of the composition, preferably between 50 ppm and 20000 ppm by weight, more preferably between 2000 ppm and 8000 ppm, even more preferably between 4000 ppm and 6000 ppm.

In a first aspect, the invention relates to the use of one or more compounds preferably selected from the group that consists of menadione nicotinamide bisulfite (MNB), menadione dimethylpirymidinol bisulfite (MPB), menadione nicotinic acid bisulfite, menadione adenine bisulfite, menadione tryptophan bisulfite, menadione histidine bisulfite, menadione para aminobenzoic acid bisulfite and mixtures thereof, to obtain a biocidal effect on aquatic organisms that infest a mass of water of any kind and size. In particular, said biocidal effect is displayed as prevention of development, and/or elimination of the presence, of said infesting organisms and is preferably achieved for total concentrations of the active compound or compounds generally comprised between 0.5 ppm and 500 ppm, preferably between 0.5 ppm and 200 ppm, and even more preferably between 1 ppm and 100 ppm.

The surprising biocidal effectiveness of the compounds cited above can be used in the treatment of all water in general and in particular of fresh or salt water in reservoirs of any size, of water of pipes for civil and industrial use, and of the ballast water of ships, this last being a field in which, as mentioned earlier, effective solutions for controlling infesting organisms, both autotrophic ones (such as algae) and heterotrophic ones (such as animals), were limited. In a preferred manner, the biocidal use is applied in the treatment of ballast water used in marine practice.

In another embodiment, the biocidal use is performed by treating aquariums of any shape and size as well as artificial and natural fresh or salt water reservoirs, in which it is desirable to eradicate autotrophic or heterotrophic infesting aquatic species.

In another embodiment, the described biocidal use is extended to water of pipes for civil and industrial use.

Regardless of the type of mass of water that is treated, the infesting aquatic organism is preferably selected from the group that consists of phytoplankton organisms, dinoflagellates, diatoms, zooplankton organisms and bacteria.

The preferred derivatives of menadione in relation to use in the marine sector have no hydroxyl group bound to the naphthoquinone ring, and this is a particularly interesting aspect if one considers that most known molecules for biocidal treatment of water, and especially the molecules that are most active against *Dreissena Polimorfa* (which is the most feared organism), all have a hydroxyl substituent bonded to the aromatic nucleus. In some cases, this structural aspect has even been explicitly deemed essential for the activity of the molecule (see US 6,164,244 and US 6,340,468).

Surprisingly, it has been found instead that the absence of hydroxyl substituents not only does not compromise the effectiveness of the molecules but is perfectly compatible with a strong biocidal activity against various microorganisms, including *Dreissena Polimorfa.*

The absence of the hydroxyl substituent also allows an interesting modulation of the biocidal activity, reducing in particular the toxicity of the active ingredient on fish fauna, so as to limit the negative impact produced by unloading into the sea both treated ballast water and water from civil or industrial pipes. As a further evidence of harmlessness for fish species, it can be noted that MNB and MPB are currently administered as food integrators to species of zootechnical interest, including fish.

Another particularly advantageous aspect of the use of the derivatives of menadione and especially of the preferred ones specified above is their high water-solubility, so that it is easy to ensure a uniform concentration of active ingredient in the treated water. By contrast, the biocidal substances used so far for similar purposes are hard to dissolve in the water mass, and therefore inhibition of the target organisms is achieved only in a confined region in the vicinity of the biocide particle, whereas substantial survival of these organisms in most of the treated mass of water is to be expected.

The solubility aspect is particularly important if one considers that biocidal molecules are normally used in extremely small quantities (on the order of a few parts per million) and the quantity of liquid that must be effectively disinfested is often very large.

Moreover, in the case of active ingredients that have an acknowledged toxic activity with respect to the fish fauna, a change in their concentration as a consequence of problems of poor water-solubility of the active ingredient has significantly harmful consequences on the entire ecosystem and not only on the microorganisms to be eliminated.

Another advantage of menadione nicotinamide bisulfite (MNB), menadione dimethylpirymidinol bisulfite (MPB), menadione nicotinic acid bisulfite, menadione adenine bisulfite, menadione tryptophan bisulfite, menadione histidine bisulfite, menadione para aminobenzoic acid bisulfite and mixtures thereof is the salification of the quinone nucleus with a complex organic base instead of with a simple metal such as for example sodium (as in MSB); this aspect has allowed to overcome the problems related to possible alteration of the osmotic balance following disposal of water that has reached the end of the treatment cycle.

It should also be noted that the preferred molecules identified above have demonstrated high and entirely unexpected biodegradability, which is independent of photoexposure, a condition that US 6,164,244 instead considered essential.

The degradability of a substance is normally a factor that is impossible to predict on a structural basis. In the case of the preferred compounds used in the present invention, and of MNB and MPB in particular, the inventors have found a surprisingly positive result. On the basis of the available data related to the compounds that are chemically more similar to the derivatives of menadione described here, one could have expected a degradability comprised between the 0% (percentage calculated on the initial mass of product expressed as mg/l) of menadione (2-methyl-1,4-naphthalenedione) and the 17% (assessed after 28 days) of menadione sodium bisulfite (where the calculation was performed by using the OECD301 A guidelines annexed to EEC directive 92/69/EEC C4-A). It has instead been found unexpectedly that 7 days after the beginning of the test, the degradability of MNB and MPB was as much as eight times higher than that of MSB, while after 28 days it was 40%, a value that is even well above the 17% reported for MSB.

Therefore, it is noted that if the achieved biocidal activity is substantially equal, the compounds according to the invention have, with respect to compounds that are known for the same purpose, an unexpectedly higher degree of biodegradability and are therefore far more suitable.

The improvements in terms of biodegradability cannot be explained easily even after the fact (i.e., even after verifying them experimentally), since the most evident difference between some compounds known for similar purposes and for example MNB, MPB is only in the cationic part. Nonetheless, the variation of this portion has proved itself to have a significant and unpredictable influence on the biodegradability of the molecules as a whole.

All the derivatives of menadione are furthermore easily available, since the usefulness of their naphthoquinone nucleus in the industrial process for the synthesis of vitamin K analogues allows its quick and inexpensive synthesis. On the contrary for hydroxylated molecules, one has to deal with their reduced availability and high cost. Juglone, for example, is not commercially available in industrial amounts, but is synthesized only in small quantities for particular applications, and its extraction from walnut shells (see US 6,164,244), albeit potentially interesting, is unquestionably complicated and expensive.

In another aspect, the biocidal composition is administered to an animal, preferably an animal of zootechnical interest. Surprisingly now it has been found that menadione and its derivatives in general are capable of performing the strong and useful biocidal activity, shown in relation to the marine field, also in the zootechnical field. This activity in the veterinary field occurs preferably against pathogenic microorganisms and had never been described until now.

The biocidal activity in the zootechnical field occurs advantageously if the menadione and derivatives thereof are administered in a quantity preferably comprised between 0.50 and 750 mg of menadione/Kg of body weight/day, more preferably between 0.8 and 600 mg of menadione/Kg of body weight/day, even more preferably between 1 and 20 mg of menadione/Kg of body weight/day. Advantageously, the method of administration is such as to allow intestinal and/or rumenal release of the active components. As will be noted, the quantities cited above are expressed in milligrams of menadione; however, the power relative to menadione of its derivatives is known to the person skilled in the art, and therefore once the quantities referred to menadione are described, the respective quantities of the various derivatives can be calculated easily.

In the table of example 1, the quantities cited above as milligrams of menadione per kilogram of body weight are expressed in terms of milligrams of menadione/animal/day for a few species of zootechnical interest. Moreover, it is evident that the indicated quantities can vary in relation to contingent factors, such as the specific disease to be treated, its severity and state of progress, the age and physiological state of the treated animal, any co-presence of accessory diseases that modify the absorption or activity of the administered compounds.

The doses for use indicated by the present invention are different, and specifically higher, than those usually used if menadione and its derivatives are used as vitamin K(3) supplements (integrators). According to standard practice, in order to achieve a vitaminic effect, common doses in fact vary according to the species and are generally comprised between 0.5 and 5.0 mg/Kg of finished feed ready for animal consumption (see in this regard Lee Russell McDowell "Vitamins in Animal Nutrition" Academic Press Inc. San Diego 1989; NRC "Nutrient Requirements of Poultry, Ninth revised Edition" National Academic Press Washington D.C. 1994; INRA "L'alimentation des animaux monogastriques: porc, lapin, volailles" 2^{eme} èdition Paris 1989), or between 0.01 and 0.30 mg per kilogram of body weight per day, where the species characterized by higher doses are usually the bird species, since they are more exposed to suffering from primary or secondary vitaminic deficiencies. Example 1 shows the generic ranges of milligrams of vitamin K3 (menadione) per animal per day, administered to some animal species of zootechnical interest in order to obtain the known vitamin effect.

In a particular embodiment, the present invention therefore relates to the use of a compound selected from the group that consists of menadione and derivatives and mixtures thereof for the preparation of a medicament for the prevention and treatment, in an animal, of morbid conditions associated with an abnormal proliferation of pathogenic microorganisms.

The term "derivatives" of menadione is used to designate any chemical compound in which one or more units of menadione are recognizable. Preferred derivatives of menadione are selected from the group that consists of menadione nicotinamide bisulfite (MNB), menadione sodium bisulfite (MSB), menadione dimethylpirymidinol bisulfite (MPB), menadione nicotinic acid bisulfite, menadione adenine bisulfite, menadione tryptophan bisulfite, menadione histidine bisulfite, menadione para aminobenzoic acid bisulfite, menadione piperazine bisulfite, menadione styramine bisulfite, menadione epoxide, menadione triamino triazine bisulfite and mixtures thereof.

The term "animals" is used to designate members of all existing higher animal classes and preferably mammals and oviparous animals, preferably human beings and, among zootechnical species, ruminants and monogastric animals, even more preferably bovines, sheep, goats, swine, avicultural species and rabbits.

In a preferred embodiment, the animals are fed with the claimed substances and/or preparations thereof to prevent the proliferation of pathogenic microorganisms.

The language "pathogenic microorganism" is used to designate any microorganism capable of causing or facilitating the onset or development of a morbid condition in a living animal as defined above. The pathogenic microorganisms belong equally to prokaryotes, preferably bacteria, and to eukaryotes, preferably protozoa and helminths.

The term "antimicrobial" designates advantageously a biocidal effect against parasitic microorganisms, where the parasitic microorganisms are protozoa, bacteria and helminths.

The term "composition" designates both a preparation such as a feed which contains the active components at a medium-low concentration and is therefore ready for use, and a preparation which contains more concentrated active components and is therefore advantageously diluted with suitable excipients and ingredients that are common in the field to make it suitable for its final use. The term also designates a preparation such as for example a coated tablet which contains concentrated active ingredients but is ready for use.

In a preferred embodiment, the invention describes a new use, advantageously in particular quantities and with particular methods of administration, of menadione and derivatives thereof to obtain an antimicrobial effect against pathogenic microorganisms as defined above, wherein the antimicrobial effect is also accomplished by means of an active control on the causative agent of or the agent associated with the morbid state at issue and not only by acting at the level of the symptoms of said morbid state. Therefore, it has been found unexpectedly that menadione and its derivatives can be used advantageously for example in improving the clinical condition of coccidiosis not only because they act at the level of blood complications but also and most of all because they act directly on the protozoan agent that causes the disease, i.e., *Eimeria sspp.*

In particular, it has been found surprisingly that menadione and its derivatives have said significant antimicrobial effect against bacteria, protozoa and helminths, regardless of the type of animal treated. Therefore, the new and advantageous use of menadione and its derivatives according to the invention finds interesting and useful applications in the zootechnical field and in the human field in the prevention and treatment of bacterial, protozoan and helminthic diseases.

The antimicrobial effect, especially if performed at the bacterial level, can be associated with a control of the pathogenic intestinal flora of the treated animals, so as to have, in the zootechnical field, a further auxinic (i.e., growth-promoting) effect, hitherto obtainable only by using veterinary drugs such as Monensin or Carbadox, whose drawbacks have been discussed extensively above. The growth-promoting effect refers not only to the increase in weight and size of the treated animals but also to an improvement in food utilization, i.e., better food conversion (described technically as the conversion index, i.e., the Kg of feed required to produce one Kg of live weight).

The spectrum of bacteria that have been found to be sensitive to menadione and its derivatives in the zootechnical field has been found to be unexpectedly broader than the range already described above in relation to the use of the same compounds in the marine sector, and includes Gram-positive and Gram-negative bacteria, preferably bacteria that belong to the *Enterobacteriaceae, Pseudomonaceae, Alteromonadaceae, Vibrionaceae, Micrococcaceae* and *Bacillaceae* family, even more preferably bacteria that belong to the genera *Escherichia, Salmonella, Pseudomonas, Alteromonas, Bacillus, Corynebacterium, Vibrio, Aeromonas, Micrococcus, Staphylococcus, Campylobacter, Yersinia, Shigella* and *Clostridium,* even more preferably bacteria selected from the group that consists of *Escherichia coli, Shigella sonnei, Staphylococcus aureus, Vibrio parahaemolyticus, Bacillus subtilis* and *proteus, Salmonella enteritidis, Clostridium perfringens.* All these agents cause pathological conditions both in human beings and in animals of zootechnical interest, so as to further justify the application of the compounds according to the invention also to the field of human medicine.

The protozoa that have been found to be sensitive to treatment with menadione and its derivatives according to the invention are preferably the ones that belong to the genera *Eimeria,* the agent responsible for coccidiosis, *Histomonas* and *Trichomonas.* Even more preferably, they are the protozoa selected from the group that consists of *Eimeria brunetti, E. acervulina, E. tenella, E. necatrix, Histomonas meleagridis, Trichomonas gallinarum.*

Helminths that have been found sensitive to treatment with menadione and its derivatives according to the invention are preferably the ones that belong to the groups of Nematodes, Cestodes and Trematodes and even more preferably those selected in the genera *Haemoncus, Ascaris, Capillaria, Heterakis Echinuria, Trichostrongylus, Ostertigia.*

The antimicrobial effect, associated or not with a growth-promoting effect, is achieved preferably in humans and in bird species, swine, ruminants and rabbits.

The demonstrated biocidal activity was so high and effective that the use of menadione and its derivatives can truly be described as a real alternative in the zootechnical field to classic veterinary drugs and to other methods known in the field for the prevention and treatment of pathological conditions arising from the proliferation of parasitic or saprophytic organisms, be they bacteria, protozoa or intestinal worms.

In a different aspect, the present invention furthermore relates to a composition having an essentially antimicrobial activity, which comprises a compound selected from the group that consists of menadione and derivatives and mixtures thereof, said compound being present in a total quantity comprised between 10 ppm and 250000 ppm by weight of menadione, preferably between 50 ppm and 20000 ppm, more preferably between 2000 ppm and 8000 ppm, even more preferably between 4000 ppm and 6000 ppm. The quantities are expressed by weight of menadione on the total weight of the composition.

Antimicrobial activity, menadione derivatives and preferred derivatives are defined as above in relation to the biocidal use in the zootechnical field.

The compositions according to the invention have proved themselves effective in achieving an antiprotozoan and/or antibacterial and/or anthelmintic effect, optionally accompanied by a growth-promoting effect on animals, preferably human beings and bird species, swine, ruminants and rabbits.

In a particularly advantageous embodiment, the compositions according to the invention comprise the active compounds in combination with at least one agent selected from the group that consists of:
a) edible excipients for food and zootechnical premixtures,
b) food and zootechnical additives,
c) organic and/or inorganic acidifiers,
d) amino acids, vitamins and mineral salts,
e) powdered or reconstituted whey and milk,
f) vegetable extracts and/or essential oils or preparations thereof,
g) live or freeze-dried and/or attenuated bacterial cultures, preferably of sporogenous and/or heat-resistant bacteria,
h) lysates of bacteria or protozoa,
i) pharmaceutically acceptable carriers and excipients, and
l) stabilizing agents or antioxidants, for support.

If one or more of the additives listed above are present, menadione and its derivatives or mixtures thereof are advantageously present in the composition preferably in an amount comprised between 10 ppm and 1000 ppm, more preferably between 20 ppm and 500 ppm. The quantities are expressed by weight of menadione on the total weight of the composition.

The compositions according to the invention can be in solid, liquid or semisolid form, essentially depending on the type and quantity of optional ingredients used for their preparation.

A particularly preferred composition comprises at least one of the optional ingredients selected from the group that consists of:
e) powdered or reconstituted whey and milk,
f) vegetable extracts and/or essential oils or preparations thereof,
g) live or freeze-dried and/or attenuated bacterial cultures, preferably of sporogenous and/or heat-resistant bacteria,
h) lysates of bacteria or protozoa.

A particularly preferred composition comprises the stabilizing or support agents in combination with edible excipients for zootechnical food additives or pharmaceutically acceptable carriers and excipients or even more preferably both types of excipient.

In a preferred embodiment, the composition according to the invention is in the form of a food additive for zootechny. In this case, one or more edible excipients typical of this field are also advantageously comprised in order to formulate optimally the menadione and its derivatives.

In another embodiment, the composition according to the invention is in the form of a pharmaceutical formulation having antimicrobial activity for zootechnical or human use, wherein the menadione and its derivatives are present in combination with one or more pharmaceutically acceptable carriers and excipients.

Menadione and its derivatives have a detectable auxinic and therapeutic activity regardless of the method of administration. However, since the compounds according to the invention are essentially administered orally, it has been found that their activity is optimized if they are formulated so as to withstand the acid degradation that occurs at the level of the gastric system of animals. Preferred pharmaceutically acceptable carriers and excipients are therefore the ones known in the sector to produce a modified release of the active ingredient both in terms of time (prolonged or delayed release) and in terms of location (for example gastroresistant formulations based on hydrogenated fatty acids).

The final effect is therefore an increase in the power of the compounds at the intestinal level. Therefore, in a highly preferred embodiment the compositions according to the invention are formulated so that the active compounds that they contain are resistant to gastric and rumenal degradation.

In the preferred embodiments in which the compositions are coated, menadione and its derivatives are coated with methods similar to technologies for pelletizing or coating solid pharmaceutical forms with an emulsion of fatty acids, preferably hydrogenated fatty acids, lipids, carboxymethylcellulose (CMC), cyclodextrins, lignosulfonates taken individually or in a mixture. Likewise, it is possible to coat the menadione and its derivatives with gastro- or rumen-resistant matrices. The coated compositions are used in particular for the preparation of food additives and medicaments.

Menadione and its derivatives can also be combined with one or more stabilizing or supporting agents that are known for this purpose, such as silica, silica gel, clays and mixtures thereof, styrene resins.

The use of highly concentrated compositions, such as compositions comprising menadione or its derivatives in concentrations from 10 ppm to 250000 ppm, is justified by the fact that the person skilled in the art will understand immediately that the quantities of menadione and derivatives expressed on the weight of the compositions that comprise them can undergo considerable fluctuations depending on how said compositions are prepared and administered by the end user, always maintaining, however, the antimicrobial purpose described and claimed here for the first time.

For example, if the active components are present in concentrations for example between 50 ppm and 20000 ppm, more preferably between 2000 ppm and 8000 ppm, even more preferably between 4000 ppm and 6000 ppm by weight of menadione or derivative thereof on the total weight of the composition, the preparation can be advantageously a concentrated formulation, which the end user will have to dilute so as to make it suitable for direct administration to the animal (for example as feed), or could also be a form of concentrated administration meant for direct use and taken from the pharmaceutical field, such as for example tablets, coated tablets, powders, microencapsulated solutions, or other suitable forms.

Likewise, if the active components are present for example at a concentration between 10 ppm and 1000 ppm, preferably between 20 ppm and 500 ppm by weight of menadione or derivative thereof on the weight of the composition, the formulation can be an edible zootechnical preparation or feed, in which the concentrations of the active compounds are lower than in the previous case, since the formulation is prepared with the prospect of direct administration to animals without further dilutions.

Further characteristics and advantages of the present invention will become better apparent from the description of the following preferred embodiments, intended exclusively as non-limiting examples.

### Example 1

Table 1 lists, for ten different species, the quantities of menadione and its derivatives expressed as equivalent mg of vitamin K3 currently in use to obtain the known vitamin-like effect and quantities suitable to obtain instead the antimicrobial effect according to the invention (an indicative average weight and weight range per animal is also listed for each species).

**Table 1**

| Species | Average weight | Vitamin use (mg K3) | Antimicrobial use (mg K3) |
|---|---|---|---|
| Rabbit | 2.4 Kg (2.0-2.8) Kg | 0.2-0.3 mg/animal/day | 5-1500, preferably 10-45 mg/animal/day |
| Chicken | 2 Kg (1.7-2.2) Kg | 0.4-0.6 mg/animal/day | 4-1300, preferably 8-36 mg/animal/day |
| Laying hen | 2.25 Kg (2.0-2.5) Kg | 0.5-0.6 mg/animal/day | 5-1200, preferably 10-36 mg/animal/day |
| Turkey | 12.5 Kg (10-15) Kg | 0.8-1.0 mg/animal/day | 10-2500, preferably 20-75 mg/animal/day |
| Piglets | 22.5 Kg (20-25) Kg | 1.0-2.0 mg/animal/day | 25-10000, preferably 50-300 mg/animal/day |
| Swine | 110 Kg (100-120) Kg | 1.5-2.5 mg/animal/day | 75-25000, preferably 150-750 mg/animal/day |
| Veals | 135 Kg (70-200) Kg | 1.5-6.0 mg/animal/day | 75-60000, preferably 150-1800 mg/animal/day |
| Bullocks | 350 Kg (200-500) Kg | 0 mg/animal/day | 3500-120000, preferably 2400-3600 mg/animal/day |
| Sheep | 85 Kg (50-120) Kg | 0 mg/animal/day | 75-25000, preferably 150-750 mg/animal/day |
| Human being | 72.5 Kg (70-75) Kg | 0 mg/animal/day | 35-10000, preferably 100-500 mg/animal/day |

### Example 2

The effect of menadione nicotinamide bisulfite on *Haemoncus contortus* is assessed by measuring the motility of the larvae (L1), Table 2, and the faecal count of the larvae (L3), Table 2.1, according to the methodology described in Fetterer R. et Al. (1989) Vet. Parasitol. 32:181-189.

**Table 2.1**

| | | | | |
|---|---|---|---|---|
| µg of MNB/ml | 0 | 1 | 10 | 100 |
| % of motile larvae (L1) | 84 | 70 | 43 | 3 |

**Table 2.2**

| | | | | |
|---|---|---|---|---|
| µg of MNB/ml | 0 | 1 | 10 | 100 |
| Total no. of larvae (L3) | 2000 | 650 | 310 | 35 |

The effect of menadione nicotinamide bisulfite is evident and substantially proportional to the administered dose. In particular, at the dose of 100 µg/ml the effect observed on the motility of the larvae and on the faecal count is significant.

### Example 3

The effect of a preparation comprising menadione sodium bisulfite coated with saturated vegetable fatty acids on rabbits in the fattening stage is evaluated.

Composition of the preparation (per Kg of final preparation):
-- MSB coated with hydrogenated vegetable fatty acids (1:1 inclusion ratio) 250 g;
-- colloidal silica: 30 g;
-- calcium propionate: 200 g;
-- carob flour: q.s. to 1000 g.

This preparation is added in the quantity of 1 Kg per ton of feed, so that the final concentration of MSB is equal to 125 ppm/Kg of finished food. Since rabbits are fed with 120-150 grams of feed/day, the concentration of MSB is equal to an average daily dose of approximately 15-20 mg of MSB/animal per day.

New Zealand White animals, weaned at 32 days, were grown with the preparation cited above and compared with animals of the same race, fed with the same basic feed but treated with bacitracin zinc in a quantity equal to 150 mg/100 Kg of feed and with animals lacking any drug integration.

The data of the table clearly indicate that at the end of the fattening cycle (i.e., 53 days after weaning), the MSB produced results comparable to, or even better than, those of bacitracin zinc, both as regards the growth of the animals and most of all as regards the mortality percentage. Vice versa, the animals that did not receive any medicinal preparation reported a lower conversion and higher mortality due mostly to intestinal forms (Clostridium sp.)

**Table 3**

| | Control | Bacitracin zinc 1.5 ppm | MSB 250 ppm |
|---|---|---|---|
| Initial number of animals | 200 | 200 | 200 |
| Average initial weight (grams) | 810 | 820 | 817 |
| Final number of animals | 165 | 185 | 191 |
| Final average weight (kilograms) | 2.65 | 2.77 | 2.84 |
| Average daily growth (grams) | 34.7 | 36.8 | 38.1 |
| Mortality % | 16 | 7.5 | 4.5 |

### Example 4

The table below lists the results of the toxicity tests performed on fish fauna and on some phyto- and zooplankton species considered among the possible targets of the claimed method. In particular, a comparison was made between the LC₅₀ of MNB and MPB and those of some compounds that can be used for the same purpose. For example, hydroxylated molecules such as 2-methyl-5-hydroxy-1,4-naphthalenedione and juglone, as well as some molecules mentioned in the previously cited documents, such as 2-methyl-1,4-naphthalenedione and 2-methyl-anthraquinone, were considered.

**Table 4**

| LC₅₀ (ppm) | *Isochris Galbana* | *Dreissena Polimorfa* Larvae | *Eurytemora Affinis* | *Artemia Salina* | *Rainbow trout* (assessed on 96h) |
|---|---|---|---|---|---|
| MNB | 0.5 | 1 | 5 | 5 | 0.4 |
| MPB | 0.5 | 1 | 5 | 5 | 0.4 |
| 2-methyl-1,4-naphthalenedione | 0.05 | 500 | 5 | 5 | 0.1 |
| 2-methyl-5-hydroxy-1,4-naphthalenedione | 0.5 | 0.2 | 5 | 5 | N.A./ |
| 2-methylanthraquinone | 0.5 | 200 | 5 | 5 | N.A. |
| Juglone | / | 0.36-2.8 | / | / | 0.02 (assessed on Fathead Minnow) |

Attention is called to the great variability in the effectiveness on *Dreissena Polimorfa.* In particular, the impact of the presence of the hydroxyl group is evident from the values reported for 2-methyl-5-hydroxy-1,4-naphthalenedione (LC₅₀ equal to 0.2 ppm, very close to that of juglone) and for 2-methyl-1,4-naphthalenedione (LC₅₀ equal to 500 ppm, a value 2500 times higher). This trend is also confirmed by 2-methyl-anthraquinone (LC₅₀ equal to 200 ppm).

Although there is intention to remain bound to definitive hypotheses, it is in any case possible to hypothesize that the possible explanations for this behaviour may include an insufficient distribution and uniformity of the active ingredient, especially in view of the insolubility in water of 2-methylquinones.

The only exceptions to this generalized trend are constituted by MNB and MPB, which exhibit a biocidal activity against the *Dreissena Polimorfa* mollusk that is compatible with their large-scale use.

These molecules have an excellent biocidal activity for most of the organisms considered; said activity is in any case comparable to the activity of quinones having an OH group in position 5 without however sharing with them high aggressiveness against fish.

### Example 5

The second fundamental factor to be considered next to biocidal activity is the degradability of the various molecules.

The hydrolysis test performed in darkness shows that MNB and MPB are rapidly degradable to a pH that is compatible with the environmental conditions.

**Table 5.1**

| ½ LIFE | Juglone | Menadione | MNB | MPB |
|---|---|---|---|---|
| pH 9 | 15 h | 550 h | < 3 h | < 3h |
| pH 6.8 | > 288 h | 1500 h | 48 h | 48 h |

In a fully similar manner, the same behaviour is confirmed if one assesses the photodegradability in sunlight and UV light (the radiation test was performed on a 10-ppm solution in drinking water (pH 6.5-7.5)

**Table 5.2**

| Light exp. | Juglone | Menadione | MNB | MPB |
|---|---|---|---|---|
| ½ life | N.A. | 1500 h | 10 h | 10 h |
| Total Degr. | 48 h | > 5000 h | 48 h | 48 h |

Finally, biodegradability according to the 92/69/EEC C4-A regulation was also assessed. The data clearly show that both MNB and MPB have a distinctly better biodegradability than 2-methyl-1,4-naphthalenedione and most of all than MSB.

**Table 5.3**

| % biodegr. OECD 301 A | Juglone | Menadione | MNB | MPB | MSB |
|---|---|---|---|---|---|
| after 7 days | N.A. | 0 | 37 | 37 | 5 |
| after 14 days | N.A. | 0 | 37 | 37 | 10 |
| after 28 days | N.A. | 0 | 40 | 40 | 17 |

Therefore, from this point of view MNB and MPB have evident advantages with respect to the use of any compound chemically correlated to them, since a better OECD 301 biodegradability (and more generally a better degradability to light and as a function of pH) lead to the expectation of a distinctly shorter average life when discharged into the sea together with ballast water and therefore a lower danger level for the fish fauna.

### Example 6

As noted repeatedly, the water solubility of the biocidal candidate is an essential requirement for advantageous use in biological water control. Accordingly, this property was evaluated for four of the compounds that are representative of the chemical classes to which the biocides currently in use belong. (The water-solubility of juglone was evaluated without the aid of adjuvant organic solvents such as alcohols or acetone)

**Table 6**

| Sol. in H₂O (gr/l at 20°C) | Juglone | Menadione | MNB | MPB |
|---|---|---|---|---|
| | Ins. | ins. | 19 | 10 |

The results summarized in the above table clearly show the difficulties that would be encountered if one decided to utilize the biocidal properties of molecules such as juglone and menadione but one did not want to (or could not) resort to a premixing of these compounds in suitable solvents before addition in the mass of water to be treated.

At the doses compatible with any possible use according to the present invention, and in particular if the water to be treated is ballast water (in which case the dosage would be a few ppm), it is in fact difficult to ensure uniform distribution of the active ingredient in the water; a particle of the active ingredient can for example be effective in its immediate vicinity but harmless for most of the mass of water.

The density of the particle also can have an important role in this regard: in particular, if it were denser than the water it would settle to the bottom, while if it were less dense it would float on the surface. In any case, uniform distribution appears to be impossible.

### Example 7

The MNB compound was tested in particular for its antibacterial activity on pathogenic and non-pathogenic organisms. The biocidal activity of MNB was estimated by using two separate methods: the agar diffusion test (Kirby-Bauer method) and the determination of the Minimum Inhibiting Concentration (MIC) and the Minimum Bactericidal Concentration (MBC).
a) Kirby-Bauer test performed after 24 hours in darkness.
In this test, paper disks impregnated with known quantities of MNB are placed on the surface of agar disks injected at confluence with a standard suspension of a bacterial strain. The antibacterial agent diffuses on the plate, causing the formation of an area of inhibition of the growth of the bacterial strain around the disk with the inhibiting quantity of the agent. By virtue of a calibration curve it was possible to classify the isolates into susceptible, moderately resistant, and resistant.
The method being considered, therefore, assesses the effectiveness of a single quantity of agent (MNB 100 mg) against two control strains of Gram-positive and Gram-negative aerobic bacteria (Gram-negative: Pseudomonas elongata NCIMB 1141; Gram-positive: Staphylococcus aureus ATCC 25923).
The results are given in the table and represent the size of the inhibition halos in millimetres after contact with 100 µg of MNB.

**Table 7.1**

| | |
|---|---|
| Bacterial strain being tested | MNB (100 µg) |
| Pseudomonas elongata | 20 mm |
| Staphylococcus aureus | 20 mm |

b) Test for determining the MIC (minimum inhibiting concentration) and the MBC (minimum bactericidal concentration) of MNB with respect to pathogenic organisms after the colonies were kept in darkness for 24 hours. Tested concentration range: 0.12-64 mg/l. The tested strains were environmental isolates identified by means of an internal number or were standard strains identified by means of the ATCC or MNCIB filing number. The results are summarized in Table 7.2.

**Table 7.2**

| Strain | MIC (mg/l) | MBC (mg/l) |
|---|---|---|
| Pseudomonas aeruginosa ATCC 10145 | >64 | >64 |
| Pseudomonas elongata NCIMB 1141 | 8 | 16 |
| Bacillus subtilis ATCC 25923 | 12 | 32 |
| Staphylococcus aureus ATCC 25923 | 8 | 16 |
| Strain | MIC (mg/l) | MBC (mg/l) |
| Escherichia coli ATCC 25922 | >64 | >64 |
| Pseudomonas putida NCIMB 1960 | >64 | >64 |
| Bacillus licheniformis NCIMB 1525 | 16 | 32 |
| Aeromonas sp.CH34 | 32 | 64 |
| Micrococcus sp. AE11 | 8 | 16 |
| Vibrio parahaemolyticus AD19 | 1 | 4 |
| Corynebacterium sp. CT15 | 8 | 16 |
| Vibrio sp. AD18 | 16 | 32 |
| Vibrio sp. CH45 | 16 | 16 |
| Micrococcus sp. AD10 | 16 | 32 |
| Vibrio sp. AE7 | 16-32 | 32-64 |

### Example 8

The effect of a preparation comprising menadione dimethylpirymidinol bisulfite (MPB) on coccid infection in chicken was assessed.

Composition of the preparation (per Kg of final preparation):
-- MPB coated with vegetable hydrogenated fatty acids (1:1 inclusion ratio) 500 g,
-- Vitamin concentrate 300 g,
-- Soybean meal 200 g,
where the expression "vitamin concentrate" designates the following composition per Kg: Vit A. 50,000,000 IU, Vit D3 10,000,000 IU, Vit E 75 g, Vit K3 20 g, Vit B1 10 g, Vit B2 30 g, Vit B6 20 g, Vit B12 0.1 g, calcium pantothenate (D) 60 g, nicotinic acid 200 g, folic acid 5 g.

This preparation was added in the amount of 1 Kg per ton of feed, so that the final concentration of MPB was equal to 250 ppm of MPB (equivalent to approximately 109 ppm of menadione). Since a chicken eats on average 70-80 grams of feed per day, the daily dose of MPB was equal to approximately 17-20 mg/animal per day.

The chickens (Ross males) were raised in individual cages for four weeks with a commercial feed in comparison with the same feed integrated with said preparation at the dose of 1 Kg/ton.

At the end of the third week, the chickens were weighed and divided into uniform weight groups within each treatment and were orally inoculated (1 ml) respectively with 50,000 oocysts of Eimeria tenella or 100,000 oocysts of E.acervulina. The fecal count of the oocysts was performed (method described in Hodgson J.N. 1970, Exp. Parasitol. 28:99-102) 16 hours before killing the animals.

The data of the table show that with respect to a feed that contains no anticoccidic substance, the administration of MPB produced a substantial and statistically significant reduction in the emission of oocysts. (a) P= 0.05, n=6

**Table 8.1**

| Excretion of oocysts | Eimeria acervulina (x 10⁸) | Eimeria tenella (x 10⁸) |
|---|---|---|
| Control | 31.3 ± 0.5 | 2.8 ± 0.1 |
| Control + MPB | 5.9 ± 0.8^{a} | 0.44 ± 0.05^{a} |

The coccidiostatic effect is confirmed by the score of the lesions six days after inoculation (method described by Johson J. et Al. 1970, Exp.Parasitol. 28:30-36).

**Table 8.2**

| Lesion score | Eimeria acervulina (Eliminare x 10⁸) | Eimeria tenella (Eliminare x 10⁸) |
|---|---|---|
| Control | 2.85 ± 0.22 | 2.70 ± 0.31 |
| Control + MPB | 1.88 ± 0.25^{a} | 1.48 ± 0.21^{a} |

Although only some preferred embodiments of the invention have been described in the text, the person skilled in the art will understand immediately that it is in any case possible to obtain other equally advantageous and preferred embodiments.

The disclosures in Italian Patent Application No. MI2003A001011, in USSN 10/740,396 and in Italian Patent Application No. MI2004A000678 from which this application claims priority are incorporated herein by reference.

## Claims

1. The use of at least one quinone compound selected from the group that consists of menadione and derivatives and mixtures thereof for the production of a biocidal composition for preventing the growth, or eradicating the presence, of at least one microorganism from a substrate selected between a mass of water and an animal,
provided that when the use is in a mass of water, the at least one quinone compound is not menadione, menadione sodium bisulfite (MSB), menadione piperazine bisulfite, menadione styramine bisulfite, menadione epoxide, menadione triamino triazine bisulfite, and mixtures thereof.

2. The use according to claim 1, **characterized in that** the biocidal preparation is a medicament for the prevention and treatment, in an animal, of morbid conditions associated with an abnormal proliferation of pathogenic microorganisms.

3. The use according to claim 1, **characterized in that**:
-- the biocidal composition is added to a mass of water at risk of contamination or already contaminated by said microorganism,
-- said quinone compound is selected from the group that consists of menadione nicotinamide bisulfite (MNB), menadione dimethylpirymidinol bisulfite (MPB), menadione nicotinic acid bisulfite, menadione adenine bisulfite, menadione tryptophan bisulfite, menadione histidine bisulfite, menadione para aminobenzoic acid bisulfite, and
-- said microorganism is an infesting aquatic organism selected from the group that consists of phytoplankton organisms, dinoflagellates, diatoms, zooplankton organisms and bacteria.

4. The use according to claim 2, **characterized in that** the animals are mammals and oviparous animals.

5. The use according to claim 2, **characterized in that** the pathogenic microorganisms are selected from the group that consists of bacteria, protozoa and helminths.

6. The use according to claim 5, **characterized in that** the bacteria are selected from the group that consists of bacteria that belong to the *Enterobacteriaceae, Pseudomonaceae, Alteromonadaceae, Vibrionaceae, Micrococcaceae* and *Bacillaceae* family.

7. The use according to claim 5, **characterized in that** the protozoa are selected from the group that consists of protozoa that belong to the genus *Eimeria, Histomonas* and *Trichomonas.*

8. The use according to claim 5, **characterized in that** the helminths are selected from the group that consists of Nematodes, Cestodes and Trematodes.

9. The use according to claim 2, **characterized in that** the menadione and derivatives and mixtures thereof are administered in a total quantity comprised between 0.50 and 750 mg of menadione/Kg of body weight/day.

10. The use according to claim 2, **characterized in that** the biocidal composition comprises menadione and derivatives or mixtures thereof in a total quantity comprised between 10 ppm and 250000 ppm by weight of menadione on the total weight of the composition, preferably between 50 ppm and 20000 ppm, more preferably between 2000 ppm and 8000 ppm, even more preferably between 4000 ppm and 6000 ppm.

11. The use according to claim 10, **characterized in that** the total quantity of menadione and derivatives thereof is comprised between 10 ppm and 1000 ppm, preferably between 20 ppm and 500 ppm, by weight of menadione on the total weight of the biocidal composition, and said biocidal composition furthermore comprises at least one agent selected from the group that consists of:
a) edible excipients for food and zootechnical premixtures,
b) food and zootechnical additives,
c) organic and/or inorganic acidifiers,
d) amino acids, vitamins and mineral salts,
e) powdered or reconstituted whey and milk,
f) vegetable extracts and/or essential oils or preparations thereof,
g) live or freeze-dried and/or attenuated bacterial cultures, preferably of sporogenous and/or heat-resistant bacteria,
h) lysates of bacteria or protozoa,
i) pharmaceutically acceptable carriers and excipients, and
l) stabilizing agents or antioxidants, for support.

12. The use according to claim 3, **characterized in that** said mass of water to be treated is selected from the group that consists of:
-- water used as ballast in marine practice,
-- fresh or salt water of reservoirs of any size,
-- water of pipes for civil and industrial use, and
-- fresh or salt water of aquariums of any shape and size.

13. The use according to claim 3, **characterized in that** said phytoplankton organisms are algae.

14. The use according to claim 3, **characterized in that** said zooplankton organisms are selected from the group that consists of copepods, crustaceans, polychaetes, mollusks and their larvae.

15. The use according to claims 13 and 14, **characterized in that** said phytoplankton and zooplankton organisms are selected from the group that consists of *Dreissena Polimorfa, Isochris Galbana, Eurytemora Affinis* and *Artemia salina.*

16. The use according to claim 3, **characterized in that** said bacteria are selected from the group that consists of bacteria that belong to the family of *Enterobacteriaceae, Pseudomonaceae, Alteromonadaceae* and *Bacillaceae.*

17. The use according to claim 16, **characterized in that** said bacteria are selected from the group that consists of bacteria that belong to the genera *Escherichia, Salmonella, Pseudomonas, Alteromonas, Clostridium, Shigella* and *Proteus.*

18. The use according to claim 3, **characterized in that** the quinone compound is menadione nicotinamide bisulfite (MNB).

19. The use according to claim 3, **characterized in that** said compound to be added to the mass of water is used in a quantity comprised between 0.5 ppm and 500 ppm, preferably between 0.5 ppm and 200 ppm, more preferably between 1 ppm and 100 ppm.

20. A composition having antimicrobial activity, comprising a compound selected from the group that consists of menadione and derivatives and mixtures thereof, said compound being present in a total quantity comprised between 10 ppm and 250000 ppm by weight of menadione on the total weight of the composition, preferably between 50 ppm and 20000 ppm, more preferably between 2000 ppm and 8000 ppm, even more preferably between 4000 ppm and 6000 ppm.

21. The composition according to claim 20, furthermore comprising at least one agent selected from the group that consists of:
a) edible excipients for food and zootechnical premixtures,
b) food and zootechnical additives,
c) organic and/or inorganic acidifiers,
d) amino acids, vitamins and mineral salts,
e) powdered or reconstituted whey and milk,
f) vegetable extracts and/or essential oils or preparations thereof,
g) live or freeze-dried and/or attenuated bacterial cultures, preferably of sporogenous and/or heat-resistant bacteria,
h) lysates of bacteria or protozoa,
i) pharmaceutically acceptable carriers and excipients, and
l) stabilizing agents or antioxidants, for support.

22. The composition according to claim 21, **characterized in that** the total quantity of menadione and derivatives thereof is comprised between 10 ppm and 1000 ppm, preferably between 20 ppm and 500 ppm, by weight of menadione on the total weight of the composition.

23. The composition according to claim 21, comprising:
-- at least one of the optional ingredients e) to h), or
-- the optional ingredient l) in combination with at least one of a) and i), or
-- all the optional ingredients a), i) and l).

24. The composition according to claim 21, **characterized in that** the stabilizing or support agents are selected from the group that consists of silica, silica gel, clays, styrene resins and mixtures thereof.

25. The composition according to claim 21, **characterized in that** said pharmaceutically acceptable carriers and excipients produce a release of the active ingredient that is modified in terms of time and/or site.

26. The composition according to claim 25, **characterized in that** said carriers and excipients are selected from the group that consists of fatty acids, lipids, carboxymethylcellulose (CMC), cyclodextrins, lignosulfonates and mixtures thereof.

27. A food additive for zootechny, comprising the composition according to claim 20 and one or more edible excipients.

28. The invention according to any one of claims 2 and 20, **characterized in that** the derivatives of menadione are selected from the group that consists of menadione nicotinamide bisulfite (MNB), menadione sodium bisulfite (MSB), menadione dimethylpirymidinol bisulfite (MPB), menadione nicotinic acid bisulfite, menadione adenine bisulfite, menadione tryptophan bisulfite, menadione histidine bisulfite, menadione para aminobenzoic acid bisulfite, menadione piperazine bisulfite, menadione styramine bisulfite, menadione epoxide, menadione triamino triazine bisulfite, and mixtures thereof.
